(19) Europäisches Patentamt
European Patent Office
Office européen des brevets

(11) Publication number: **0 083 189**
**A2**

(12) # EUROPEAN PATENT APPLICATION

(21) Application number: 82306791.3

(22) Date of filing: 20.12.82

(51) Int. Cl.[3]: C 07 C 120/14
C 07 C 121/32, B 01 J 23/88

(30) Priority: 28.12.81 JP 209950/81

(43) Date of publication of application:
06.07.83 Bulletin 83/27

(84) Designated Contracting States:
DE FR GB

(71) Applicant: Ube Industries, Ltd.
12-32, Nishihonmachi 1-chome
Ube-shi, Yamaguchi-ken(JP)

(72) Inventor: Ohdan, Kyoji c/o UBE INDUSTRIES, LTD.
Central Research Laboratory 1978-5, Oaza Kogushi
Ube-shi Yamaguchi-ken(JP)

(72) Inventor: Asada, Horoyuki c/o UBE INDUSTRIES, LTD.
Central Research Laboratory 1978-5, Oaza Kogushi
Ube-shi Yamaguchi-ken(JP)

(72) Inventor: Hidaka, Mikio c/o UBE INDUSTRIES, LTD.
Central Research Laboratory 1978-5, Oaza Kogushi
Ube-shi Yamaguchi-ken(JP)

(72) Inventor: Kurafuji, Toshio c/o UBE INDUSTRIES, LTD.
Central Research Laboratory 1978-5, Oaza Kogushi
Ube-shi Yamaguchi-ken(JP)

(72) Inventor: Oda, Yoshihiko c/o UBE INDUSTRIES, LTD.
Central Research Laboratory 1978-5, Oaza Kogushi
Ube-shi Yamaguchi-ken(JP)

(74) Representative: Arthur, Bryan Edward et al,
Withers & Rogers 4 Dyer's Buildings Holborn
London EC1N 2JT(GB)

(54) **Process for preparation of unsaturated nitriles.**

(57) Olefins are catalytically ammoxidized to the corresponding unsaturated nitriles at an elevated temperature in the vapor phase. The catalyst used has a composition of the formula:

$$Mo_aBi_bPb_cTl_dX_eA_fB_gO_h$$

wherein Mo is molybdenum, Bi is bismuth, Pb is lead, Tl is thallium, X is at least one element selected from phosphorus, arsenic and antimony, A is an alkali metal, B is at least one element selected from nickel, cobalt and tungsten, O is oxygen, and a = 10, b = 0.1 – 10, c = 0.1 – 10, d = 0.01 – 1, e = 0 – 10, f = 0 – 1, g = 0 – 10 and h = 3.15 – 112.

EP 0 083 189 A2

Croydon Printing Company Ltd.

0083189

# PROCESS FOR PREPARATION OF UNSATURATED NITRILES

## BACKGROUND OF THE INVENTION

### (1) Field of the Invention

This invention relates to an improved process for ammoxidizing an olefin such as propylene or isobutylene at an elevated temperature in the vapor phase in the presence of a catalyst to prepare a corresponding unsaturated nitrile such as acrylonitrile or methacrylonitrile.

### (2) Description of the Prior Art

Various catalysts have been heretofore proposed for use in the catalytic ammoxidation of olefins to produce unsaturated nitriles at an elevated temperature in the vapor phase.

For example, U.S. Patent No. 2,904,580 discloses a catalyst of the Mo-Bi-(P) system, U.S. Patent No. 3,688,002 discloses a catalyst comprising $BiSbO_4$ and $PbMoO_4$ , Japanese Examined Patent Publication No. 6,649/76 discloses a catalyst of the Tl-(P)-Mo-Fe-Bi-X-(Y) system in which X is selected from Ni, Mg, Co and Mn and Y is selected from Be, Ca, Zn, Sr, Cd, Sn, Cr and Pb, and Japanese Unexamined Patent Publication No. 110,997/79 discloses a catalyst of the Mo-W-Bi-Pb system. Furthermore, U.S. Patent No. 4,212,776 and U.S. Patent No. 4,148,757 disclose processes for the preparation of multi-component oxide complex catalysts of the Mo-Bi system.

In case of catalysts which are excellent in the selectivity to the unsaturated nitrile, however, in order to maintain the selectivity at a high level, it is ordinarily necessary to carry out the ammoxidation reaction while controlling the conversion of the olefin at a low level. If it is intended to maintain the conversion at a high level, the selectivity to the unsaturated nitrile tends to decrease.

Some of the conventional catalysts are capable of providing unsaturated nitriles at relatively high selectivities. However, catalysts capable of providing unsaturated nitriles at high selectivities even if the conversion is elevated to a very high level, for example, about 100%, have hardly been proposed. Since the prices of olefins have recently been increasing, it is eagerly desired in the art from the industrial viewpoint to develop a catalyst capable of forming an unsaturated nitrile at a high selectivity in a high yield even if the conversion of an olefin is maintained at a high level.

Some proposals have been heretofore made wherein catalysts comprising lead as a catalytic component are used for the ammoxidation of olefins to unsaturated nitriles. Japanese Unexamined Patent Publication No. 110997/79 discloses a catalyst of the Mo-W-Bi-Pb system, and in the working examples of this reference, it is taught that the selectivity to acrylonitrile is about 80% when the conversion is approximately 100%. Furthermore, Japanese Examined Patent Publication No. 6,649/76 teaches that a catalyst of the Tl-P-Mo-Fe-Bi-Ni-Pb system gives an acrylonitrile selectivity of 76% when the conversion of propylene is 99% (see Example 5). Moreover, U.S. Patent No. 3,688,002 teaches that a catalyst comprising $BiSbO_4$ and $PbMoO_4$ gives an acrylonitrile selectivity of 88% when the conversion of propylene is 46.1% (see Example 40).

SUMMARY OF THE INVENTION

It is primary object of the present invention to provide a catalyst of the Mo-Bi-Pb-Tl system which is capable of yielding unsaturated nitriles at a higher selectivity even if the conversion is maintained at a high level, then the conventional lead-containing catalysts.

Other objects and advantages of the present invention will be apparent from the following description.

In accordance with the present invention, there is provided an improvement in a process for the preparation of an unsaturated nitrile wherein an olefin is ammoxidized at an elevated temperature in the vapor phase in the presence of a catalyst to form the corresponding unsaturated nitrile.

The catalyst used has a composition represented by the following formula:

$$Mo_aBi_bPb_cTl_dX_eA_fB_gO_h$$

wherein Mo is molybdenum, Bi is bismuth, Pb is lead, Tl is thallium, X is at least one element selected from the group consisting of phosphorus, arsenic and antimony, A is an alkali metal, B is at least one element selected from the group consisting of nickel, cobalt and tungsten, O is oxygen, subscripts a through h are numbers expressing atomic ratios of the respective elements, and when a is 10, b is 0.1 to 10, preferably 1 to 6, c is 0.1 to 10, preferably 1 to 8, d is 0.01 to 1, preferably 0.03 to 0.5, e is 0 to 10, preferably 0.01 to 7, f is 0 to 1, preferably 0.01 to 0.5, g is 0 to 10, preferably 0.01 to 5, and h is a number satisfying the average valency of the elements other than oxygen and falling within the range of from 3.15 to 112.

DESCRIPTION OF THE PREFERRED EMBODIMENTS

The catalyst used in the present invention comprises as the main catalytic components molybdenum, bismuth, lead and thallium, and the catalytic activity of the catalyst has close relations to the proportions of these catalytic components. Ordinarily, if the proportion of bismuth to molybdenum is too small or too large, the conversion of the olefin and the selectivity to the unsaturated nitrile are reduced. If the proportion of lead to molybdenum is too small or too large, the conversion of the olefin is especially reduced and also the yield of the unsaturated nitrile is reduced. Only the catalyst having a composition represented by the above formula can produce an unsaturated nitrile at a high selectivity in a high yield even if the conversion of the starting olefin is maintained at a high level, and a preferred catalyst has a composition represented by the above general formula where the sum of the amounts of bismuth and lead is 8 to 12 gram-atoms per 10 gram-atoms of molybdenum and the atomic ratio of lead to bismuth (Pb/Bi) is in the range of from 2 to 5. Thallium

exerts a function of increasing the selectivity to the unsaturated nitrile. However, if the proportion of thallium is too small, this effect is low. If the proportion of thallium is too large, the selectivity to the unsaturated nitrile is improved, but in order to maintain a high selectivity, it is necessary to control the conversion of the olefin to a low level and the yield of the unsaturated nitrile is inevitably reduced. Accordingly, it is indispensable that the proportion of thallium should be controlled within the above-mentioned range.

The catalyst used in the present invention may contain the optional elements X, A and B. However, a catalyst comprising the elements X, A and B is especially preferred because it gives a higher yield of the unsaturated nitrile than the yield attainable by the catalyst free of these elements, though the difference of the yield is not so prominent. From the industrial viewpoint, increase of the yield of the unsaturated nitrile is very important, even though this increase is to minor extent. As the alkali metal as the element A, there can be mentioned for example, potassium, cesium, rubidium and sodium. It is preferred that the elements X, A and B be present in amounts of 0.01 to 7 gram-atoms, 0.01 to 0.5 gram-atom and 0.01 to 5 gram--atoms, respectively, per 10 gram-atoms of molybdenum.

The catalyst used in the present invention can be prepared according to the known procedures adopted for the preparation of conventional oxide catalysts.

For example, the catalyst of the present invention may be prepared according to a method in which predetermined amounts of salts and oxides of the respective catalytic components are mixed in the presence of water to form a slurry or paste and the slurry or paste is dried and then calcined; a method in which predetermined amounts of salts and oxides of the respective elements are mixed in the presence of water to form a mixed solution or suspension, the solution or suspension is evaporated to dryness and the solid is calcined; or a method in which a co-precipitate

containing the respective elements is formed from the above-mentioned mixed solution and the co-precipitate is separated from the mixed solution, dried and then calcined. Ordinarily, the calcination is advantageously carried out in an air atmosphere, and the calcination time is 0.5 to 20 hours, preferably 2 to 15 hours. The calcination temperature is 400 to 700°C, preferably 500 to 670°C. If the calcination temperature is too low, the resulting catalyst shows only a low selectivity to the unsaturated nitrile, and if the calcination temperature is too high, the resulting catalyst gives only a low conversion of the olefin. Accordingly, it is preferred that the calcination temperature in the preparation of the catalyst be controlled within the above-mentioned range.

As the starting materials of the respective elements which are used in the process for the preparation of the catalyst used in the present invention, there can be mentioned molybdenum compounds such as molybdic acid, ammonium molybdate and molybdenum oxide; bismuth compounds such as bismuth nitrate, bismuth chloride, bismuth oxide, bismuth hydroxide, bismuth hydroxynitrate, bismuth oxycarbonate, bismuth oxynitrate and bismuth oxychloride; lead compounds such as lead nitrate, lead sulfate and lead hydroxide; thallium compounds such as thallium nitrate, thallium acetate and thallium sulfate; phosphorus and phosphorus compounds such as orthophosphoric acid, metaphosphoric acid, phosphorous acid, phosphoric anhydride and various organic phosphorus compounds; arsenic and arsenic compounds such as arsenic acid, arsenic trioxide and arsenic pentoxide; metallic antimony and antimony compounds such as antimony trioxide, antimony pentoxide and antimony chloride; alkali metal compounds such as nitrates, chlorides and sulfates of alkali metals such as sodium, potassium, rubidium and cesium; nickel compounds such as nickel nitrate, nickel carbonate, nickel hydroxide and nickel oxide; cobalt compounds such as cobalt nitrate, cobalt carbonate, cobalt hydroxide and cobalt oxide; and tungsten

compounds such as ammonium paratungstate and tungstic acid. It is ordinarily preferred that ammonium salts, nitrates and oxides which are soluble in water, aqueous ammonia and nitric acid be used as the starting materials for the preparation of the catalyst. Furthermore, compounds containing two or more of the catalytic component elements, such as lead molybdate, phosphomolybdic acid and bismuth antimonate, may be used as the starting materials for the preparation of the catalyst.

The catalyst used in the present invention may be prepared according to the conventional methods described hereinbefore. However, in order to further improve the catalytic activity, it is preferred that the catalyst be prepared according to the following process.

A water-soluble molybdenum compound such as molybdic acid or ammonium molybdate is dissolved in dilute aqueous ammonia, and a compound containing the element X, such as antimony trioxide, is incorporated in the aqueous solution and the resulting liquid is mixed to obtain liquid A.

A bismuth compound soluble in nitric acid, such as bismuth nitrate or bismuth hydroxide, is dissolved in a dilute aqueous nitric acid solution, and a lead compound such as lead nitrate or lead hydroxide, a thallium compound such as thallium nitrate or thallium acetate, an alkali metal nitrate such as cesium nitrate and a compound containing the element B, such as nickel nitrate, are incorporated and dissolved in the above solution to obtain liquid B. If the compound containing the element B is ammonium tungstate or paratungstate, this compound is added to the liquid A.

The liquid B is added dropwise to the liquid A with stirring to form a liquid mixture in the form of a slurry or paste. If desired, a carrier such as silica (silica sol) or titania is incorporated into the mixture of the liquids A and B.

The pH value of the liquid mixture is adjusted to a level not higher than 2, preferably a level not higher than

about 1.5, by using, for example, an aqueous solution nitric acid solution, and the liquid mixture is aged at a temperature in the range from room temperature to 50°C with stirring for 0.5 to 5 hours.

After the pH adjustment and aging, the liquid mixture is dried by evaporation to dryness or spray drying, and is then calcined in an oxygen-containing atmosphere such as an air atmosphere, at a temperature of 400 to 700°C, preferably 500 to 670°C, for 0.5 to 20 hours, preferably 2 to 15 hours. In order to obtain a catalyst providing better results, it is preferred that the above-mentioned liquids be mixed together, the pH value of the liquid mixture be adjusted to a low level and the liquid mixture be aged, dried and then calcined. The intended catalyst is obtained from the calcination step.

In the present invention, the catalyst may be used either in the state supported on a carrier or in the unsupported state. Any of known carriers for the catalysts for the ammoxidation of olefins can be used in the present invention. For example, there can be mentioned silica, titania, alumina, silica-alumina, zirconia, diatomaceous earth, silicon carbide and various silicates. When the catalyst is supported on the carrier, the amount of the carrier is not particularly critical. However, the carrier is ordinarily used in an amount of 0.05 to 3 g, preferably 0.1 to 2 g, per g of the catalyst. The particle size and shape of the catalyst are not practically critical. The catalyst may appropriately be molded or the particle size may appropriately be adjusted depending upon the particular application conditions of the catalyst.

In the process of the present invention, the reaction is carried out in a fixed bed or a fluidized bed. In the case where the reaction is carried out in a fluidized bed, it is preferred that a carrier component such as silica sol be added at the time of the preparation of the catalyst, the mixture be spray-dried and the resulting powdery catalyst having a particle size of about 30 to about 100 μm

be used.

In carrying out the present invention, a gas substantially inactive to the ammoxidation reaction may be used as a diluent gas in addition to the starting olefin, oxygen and ammonia. As the diluent gas, there can be mentioned, for example, steam, nitrogen gas and carbon dioxide gas. Steam is especially preferred because steam has effects of improving the selectivity to the unsaturated nitrile and prolonging the catalytic activity. Accordingly, when the reaction is carried out in a fixed bed, it is especially advantageous that the reaction will be conducted in the presence of steam. Steam is added in an amount of 0.1 to 5 moles, preferably 0.5 to 4 moles, per mole of the olefin. When the reaction is carried out in a fluidized bed, water formed by the reaction exerts the above functions, and therefore, the reaction can be conducted smoothly even if steam is not particularly added.

The reaction is ordinarily carried out under atmospheric pressure. However, a slightly elevated or reduced pressure may be adopted. The reaction temperature is 350 to 550°C, preferably 420 to 500°C, and the contact time is 1.5 to 20 seconds, preferably 2 to 10 seconds. In the process of the present invention, best results can be obtained when the reaction temperature is about 450°C and the contact time is about 4 seconds.

As the olefin used in the present invention, there can be mentioned for example, propylene, isobutylene and methylstyrene. Of tnese, propylene and isobutylene are especially preferred. The olefin used need not be highly pure, but it may contain minute amounts of hydrocarbons substantially inactive to the ammoxidation reaction, such as methane, ethane and propane. It is preferred that the amount of incorporated inactive hydrocarbons be controlled to not more than 0.5 mole, especially not more than 0.1 mole, per mole of the olefin. Incorporation of hydrocarbons substantially active under the reaction conditions, such as acetylenes, should advantageously be adopted.

Pure oxygen gas may be used for the ammoxidation reaction, but an especially high purity is not required. Accordingly, from the economical viewpoint, it is preferred that air be used as oxygen.

In the present invention, the amount of oxygen fed to the olefin is 1 to 4 moles, preferably 1.2 to 3 moles, per mole of the olefin, and the amount of ammonia fed to the olefin is 0.5 to 2 moles, preferably 0.8 to 1.2 moles, per mole of the olefin. The concentration of the olefin in the starting gas supplied to the catalyst layer (comprising the olefin, oxygen, ammonia and inactive gas) is 1 to 20% by volume, preferably 2 to 10% by volume.

The present invention will now be described in detail with reference to the following Examples and Comparative Examples.

In the Examples and Comparative Examples, the conversion (%) of the olefin, the selectivity (%) to the unsaturated nitrile and the yield (%) of the unsaturated nitrile are defined as follows:

$$\text{Conversion (\%) of olefin} = \frac{\text{moles of consumed olefin}}{\text{moles of supplied olefin}} \times 100$$

$$\text{Selectivity (\%) to unsaturated nitrile} = \frac{\text{moles of produced unsaturated nitrile}}{\text{moles of consumed olefin}} \times 100$$

$$\text{Yield (\%) of unsaturated nitrile} = \frac{\text{moles of produced unsaturated nitrile}}{\text{moles of supplied olefin}} \times 100$$

Example 1

58.21 g of ammonium paramolybdate [$(NH_4)_6Mo_7O_{24}\cdot4H_2O$] was dissolved in 600 ml of an aqueous 2.5% ammonia solution maintained at 50°C, and 14.4 g of antimony trioxide ($Sb_2O_3$) was added to the solution to form a suspension. Separately, a solution formed by dissolving 81.8 g of lead nitrate [$Pb(NO_3)_3$], 0.44 g of thallium nitrate [$TlNO_3$], 0.32 g of cesium nitrate [$CsNO_3$] and 9.59 g of nickel nitrate

[$Ni(NO_3)_2 \cdot 6H_2O$] in 250 ml of warm water maintained at 50°C was mixed with a nitric acid-acidified solution formed by dissolving 39.97 g of bismuth nitrate [$Bi(NO_3)_3 \cdot 5H_2O$] in 30 ml of an aqueous 15% nitric acid solution. The obtained liquid mixture was added dropwise to the above suspension containing ammonium molybdate and antimony trioxide with stirring to obtain a slurry. Then, 200 g of 30% silica sol was added to the slurry, and then the pH value of the slurry was adjusted to 1.5 by adding thereto an aqueous 15% nitric acid soluton. Thereafter, the slurry was aged at 30°C with stirring for 3 hours.

The slurry was dried by using a drum drier to obtain a powder having a homogeneous composition. The obtained powder was heat-treated at 230°C in an air atmosphere for 16 hours, and the powder was dressed into a size smaller than 14 mesh. The powder was molded into tablets having a diameter of 5 mm and a height of 5 mm and was then calcined at 550°C in an air atmosphere for 5 hours to obtain a catalyst.

The catalyst had a composition represented by the formula $Mo_{10}Bi_{2.5}Pb_{7.5}Tl_{0.05}Sb_3Cs_{0.05}Ni_1$ (oxygen was omitted and $SiO_2$ was contained in an amount of 30% by weight).

20 ml of the so-prepared catalyst was packed in a U-shaped glass reaction tube having an inner diameter of 16 mm, and a gaseous mixture comprising propylene, ammonia, air and steam at a molar ratio of 1:1.1:11:4, respectively, was passed through the reaction tube at a flow rate of 400 ml/min. Thus, the ammoxidation of propylene was carried out at a reaction temperature of 440°C for a contact time of 3.0 seconds.

The results of the ammoxidation obtained after passage of 2 hours (the same will apply in the subsequent Examples) are shown in Table 1.

When 10 batches of the above catalyst were prepared and tested, it was found that the substantially same results as shown in Table 1 were obtained with a good

reproducibility.

Examples 2 through 8

Catalysts having a composition shown in Table 1 were prepared according to the catalyst-preparing process described in Example 1. The ammoxidation of propylene was carried out under the same conditions as described in Example 1 by using the so-prepared catalysts.

The results of the ammoxidation are shown in Table 1.

Table 1

| Example No. | Catalyst Composition* (atomic ratios) | | | | | | | Conversion (%) of Acrylonitrile | Selectivity (%) to Acrylonitrile | Yield (%) of Acrylonitrile |
|---|---|---|---|---|---|---|---|---|---|---|
| | Mo | Bi | Pb | Tl | Sb | Cs | Ni | | | |
| 1 | 10 | 2.5 | 7.5 | 0.05 | 3 | 0.05 | 1 | 99.7 | 86.6 | 86.3 |
| 2 | 10 | 2.5 | 7.5 | 0.05 | 0 | 0 | 0 | 98.1 | 83.9 | 82.3 |
| 3 | 10 | 2.5 | 7.5 | 0.05 | 3 | 0 | 0 | 99.0 | 84.6 | 83.8 |
| 4 | 10 | 2.5 | 7.5 | 0.05 | 0 | 0.05 | 0 | 96.8 | 86.8 | 84.0 |
| 5 | 10 | 2.5 | 7.5 | 0.05 | 0 | 0 | 1 | 99.0 | 84.5 | 83.7 |
| 6 | 10 | 2.5 | 7.5 | 0.05 | 3 | 0.05 | 0 | 99.3 | 85.9 | 85.3 |
| 7 | 10 | 2.5 | 7.5 | 0.05 | 3 | 0 | 1 | 99.7 | 85.4 | 85.1 |
| 8 | 10 | 2.5 | 7.5 | 0.05 | 0 | 0.05 | 1 | 99.0 | 86.3 | 85.4 |

Note * : 30% by weight of $SiO_2$ as the carrier was contained.

Comparative Example 1

A catalyst having a composition represented by the formula $Mo_{10}Bi_{2.5}Pb_{7.5}$ (oxygen was omitted and 30% by weight of $SiO_2$ was contained) was prepared in the same manner as described in Example 1 except that antimony trioxide, thallium nitrate, cesium nitrate and nickel nitrate were not used, and the ammoxidation of propylene was carried out under the same conditions as described in Example 1 by using the so-prepared catalyst.

The conversion of propylene was 98.5%, the selectivity to acrylonitrile was 77.5%, and the yield of acrylonitrile was 76.3%.

Examples 9 through 18

Catalysts having a composition shown in Table 2 were prepared according to the catalyst-preparing process described in Example 1. Phosphoric acid, arsenic acid and ammonium paratungstate were used as the phosphorus, arsenic and tungsten sources, respectively, and these compounds were added to the solution of ammonium paramolybdate as well as antimony trioxide. As the sodium, potassium, rubidium and cobalt sources, there were used nitrates of these elements, and these nitrates were dissolved in the nitric acid-acidified solution formed by dissolving bismuth nitrate in the aqueous nitric acid solution.

By using the so-prepared catalysts, the ammoxidation of propylene was carried out under the same conditions as described in Example 1 except that the reaction temperature was changed as indicated in Table 2.

The obtained results are shown in Table 2.

Table 2

| Example | Catalyst Composition* (atomic ratios) | | | | | | | | | Reaction Tempera-ture(°C) | Conversion (%) | Selectivity (%) | Yield (%) of |
|---|---|---|---|---|---|---|---|---|---|---|---|---|---|
| No. | Mo | Bi | Pb | Tl | P | As | Sb | A | B | | of Propylene | to Acrylonitrile | Acrylonitrile |
| 9 | 10 | 3 | 7 | 0.07 | 0 | 1 | 0 | Cs,0.03 | Ni,0.8 | 460 | 99.6 | 86.3 | 86.0 |
| 10 | 10 | 3.5 | 8 | 0.04 | 0.83 | 0 | 0 | Rb,0.07 | Co,2 | 470 | 99.2 | 86.9 | 86.2 |
| 11 | 10 | 2.5 | 7.8 | 0.05 | 0 | 0 | 3 | K, 0.1 | Co,1 | 450 | 99.6 | 86.4 | 86.1 |
| 12 | 10 | 2.5 | 8 | 0.05 | 1 | 0 | 0 | Na,0.08 | W, 2 | 450 | 99.9 | 86.2 | 86.1 |
| 13 | 10 | 2.5 | 7.8 | 0.1 | 0 | 0 | 3 | Cs,0.05 | Ni,0.8, W, 1 | 445 | 99.4 | 86.5 | 86.0 |
| 14 | 10 | 2.5 | 7.5 | 0.05 | 0.83 | 0 | 0 | Cs,0.05 | 0 | 440 | 98.8 | 86.1 | 85.1 |
| 15 | 10 | 2.5 | 7.5 | 0.07 | 0.83 | 0 | 0 | 0 | Ni,0.8 | 440 | 99.3 | 85.6 | 85.0 |
| 16 | 10 | 2 | 7.5 | 0.1 | 0 | 1 | 0 | K, 0.1 | 0 | 450 | 98.2 | 86.9 | 85.3 |
| 17 | 10 | 2 | 8 | 0.13 | 0 | 1 | 0 | 0 | W, 1 | 450 | 98.5 | 86.2 | 84.9 |
| 18 | 10 | 2.3 | 7.2 | 0.05 | 0.1 | 0 | 2 | Cs,0.03 | W, 2 | 440 | 99.5 | 86.8 | 86.4 |

Note * : 30% by weight of $SiO_2$ as the carrier was contained.

Example 19

By using the same catalyst as used in Example 1, the ammoxidation was carried out under the same conditions as described in Example 1 except that propylene was changed to isobutylene and the reaction temperature was changed to 420°C.

The conversion of isobutylene was 97.9%, the selectivity to methacrylonitrile was 82.5% and the yield of methacrylonitrile was 80.8%.

Comparative Example 2

By using the same catalyst as used in Comparative Example 1, the ammoxidation of isobutylene was carried out under the same conditions as described in Example 19. The conversion of isobutylene was 99.2%, the selectivity to methacrylonitrile was 77.0% and the yield of methacrylonitrile was 76.4%.

Example 20

To the slurry formed in Example 1, 380 g of silica sol was added instead of 200 g of silica sol used in Example 1, and the pH value of the slurry was adjusted to 1.5 by using an aqueous 1.5% nitric acid solution and the solution was aged with stirring for 2 hours at 30°C.

The slurry was homogenized by using a homogenizer and spray-dried by using a rotary disc type spray drier according to a conventional procedure to form fine particles. The fine powder was dried at 230°C for 16 hours and, then, heated at a temperature-elevating rate of 100°C/hr to 540°C in an air atmosphere in a calcining furnace at which temperature the fine powder is calcined for 10 hours to obtain a catalyst having an average particle size of 60 μm and having fine pores on the surface thereof. The composition of the so-obtained catalyst was the same as the composition of the catalyst obtained in Example 1, but the catalyst contained 45% by weight of silica as the carrier.

Then, 150 ml of the so-obtained catalyst was packed in a fluidized bed type reaction vessel having an inner diameter of 36 mm, and a gaseous mixture comprising propylene, ammonia, air and steam at a molar ratio of 1:1.14:12.06:1, respectively, was passed through the reaction vessel at a flow rate of 1922.3 ml/min and the ammoxidation of propylene was carried out at a reaction temperature of 470°C for a contact time of 4.68 seconds. The conversion of propylene was 99.1%, the selectivity to acrylonitrile was 87.2% and the yield of acrylonitrile was 86.4%.

Comparative Examples 3 through 13

Catalysts having a composition shown in Table 3 (30% by weight of $SiO_2$ was contained as the carrier) were prepared according to the catalyst-preparing process described in Example 1. By using these catalysts, the ammoxidation of propylene was carried out under the same reaction conditions as described in Example 1.

The obtained results are shown in Table 3.

Table 3

| Comparative Example No. | Catalyst Composition* (atomic ratios) | | | | | | | Conversion (%) of Acrylonitrile | Selectivity (%) to Acrylonitirle | Yield (%) of Acrylonitrile |
|---|---|---|---|---|---|---|---|---|---|---|
| | Mo | Bi | Pb | Tl | X | A | B | | | |
| 3 | 10 | 2 | 7.5 | 2 | 0 | 0 | 0 | 86.3 | 87.8 | 75.8 |
| 4 | 10 | 5 | 3 | 0 | 0 | 0 | W,1.5 | 98.6 | 78.1 | 77.0 |
| 5 | 10 | 4 | 4 | 0 | Sb,1 | 0 | Ni,1 | 97.5 | 79.1 | 77.1 |
| 6 | 10 | 4 | 4 | 0 | 0 | Cs,0.5 | 0 | 98.7 | 80.5 | 79.5 |
| 7 | 10 | 5 | 0 | 0.3 | 0 | 0 | 0 | 30.5 | 55.3 | 16.9 |
| 8 | 10 | 0 | 8 | 0.5 | 0 | 0 | 0 | 25.1 | 42.6 | 10.7 |
| 9 | 10 | 15 | 2 | 0.1 | 0 | 0 | 0 | 51.4 | 77.2 | 39.7 |
| 10 | 10 | 2 | 15 | 0.1 | 0 | 0 | 0 | 32.8 | 40.7 | 13.3 |
| 11 | 10 | 2 | 7.5 | 0.05 | Sb,12 | 0 | 0 | 80.0 | 73.3 | 58.6 |
| 12 | 10 | 2 | 7.5 | 0.05 | As,1 | K,2 | 0 | 59.9 | 87.0 | 52.1 |
| 13 | 10 | 2 | 7.5 | 0.05 | P,0.83 | Rb,0.1 | Ni,15 | 93.4 | 74.4 | 69.5 |

Note * : 30% by weight of $SiO_2$ was contained as the carrier.

## CLAIMS

1. A process for preparing an unsaturated nitrile wherein an olefin is ammoxidized at an elevated temperature in the vapor phase in the presence of a catalyst to form the corresponding unsaturated nitrile, characterized in that said catalyst has a composition represented by the following formula:

$$Mo_aBi_bPb_cTl_dX_eA_fB_gO_h$$

wherein Mo is molybdenum, Bi is bismuth, Pb is lead, Tl is thallium, X is at least one element selected from phosphorus, arsenic and antimony, A is an alkali metal, B is at least one element selected from nickel, cobalt and tungsten, O is oxygen, subscripts a through h are numbers expressing atomic ratios of the respective elements, and when a is 10, b is 0.1 to 10, c is 0.1 to 10, d is 0.01 to 1, e is 0 to 10, f is 0 to 1 and g is 0 to 10 and h is a number satisfying the average valency of the elements other than oxygen and falling within the range of from 3.15 to 112.

2. A process according to claim 1, wherein the subscripts a through g expressing atomic ratios of the respective elements in the catalyst are: when a = 10, b is to 6, c is 1 to 8, d is 0.03 to 0.5, e is 0.01 to 7, f is 0.01 to 0.5 and g is 0.01 to 5.

3. A process according to claim 2, wherein the sum of b and c is 8 to 12 and the ratio of c/b is 2 to 5.

4. A process according to any one of claims 1 through 3, wherein said catalyst is prepared by a process wherein salts and oxides of the respective elements are mixed in the presence of water, the aqueous mixture is dried and, then, the dried mixture is calcined at a temperature of 400 to 700°C for 0.5 to 20 hours.

5. A process according to claim 4, wherein the calcination is carried out at a temperature of 500 to 670°C for 2 to 15 hours.

6. A process according to claim 1, wherein said catalyst is supported on 0.05 to 3 g of a carrier per g of the catalyst.

7. A process according to any one of claims 1 through 6, wherein said ammoxidation of the olefin is carried out by using 1 to 4 moles of oxygen and 0.5 to 2 moles of ammonia, per mole of the olefin.

8. A process according to any one of claims 1 through 6, wherein said ammoxidation of the olefin is carried out by using 1.2 to 3 moles of oxygen and 0.8 to 1.2 moles, per mole of the olefin.

9. A process according to claim 7 or 8, wherein said ammoxidation of the olefin is carried out by using 0.1 to 5 moles of steam per mole of the olefin, in addition to the olefin, oxygen and ammonia.

10. A process according to any one of claims 1 through 9, wherein said ammoxidation of the olefin is carried out at a temperature of 350 to 550°C and a contact time of 1.5 to 20 seconds.

11. A process according to any one of claims 1 through 9, wherein said ammoxidation of the olefin is carried out at a temperature of 420 to 500°C and a contact time of 2 to seconds.

12. A process according to any one of claims 1 through 11, wherein said olefin is propylene or isobutylene.